# EUROPEAN PATENT APPLICATION

(11) **EP 4 640 264 A1**
(43) Date of publication of application: **29.10.2025**
(21) Application number: 22969140.7
(22) Date of filing: 20.12.2022
(51) Int. Cl.: A61M 25/09

(54) **GUIDEWIRE**

(71) Applicant: Asahi Intecc Co., Ltd., Seto-shi, Aichi 489-0071 (JP)
(72) Inventor: MAWATARI Nami, Seto-shi, Aichi 489-0071 (JP)
(74) Representative: Winter, Brandl - Partnerschaft mbB
(86) International application number: PCT/JP2022/046822
(87) International publication number: WO 2024/134751

(57) **Abstract**

This guidewire is provided with a core shaft made of a superelastic material, and a coil formed of a wire that has been spirally wound around the outer periphery of the distal end portion of the core shaft. A section of the core shaft positioned on an inner side of the coil has a first segment consisting of a thermally-transformed portion in which the superelastic material has been thermally transformed, and a second segment that is positioned on a rear end side of the first segment. **In** the second segment, a thermally-transformed portion having a shorter length than the first segment is formed, or no thermally-transformed portion is formed. The distal end portion of the coil has a sparsely-wound portion in which gaps are formed between portions of the wire that are adjacent to each other.

## Description

### TECHNICAL FIELD

The present invention relates to a guidewire.

### BACKGROUND ART

Medical guidewires are conventionally known that use a core shaft made of a superelastic alloy. Patent Literature 1 describes a guidewire in which a distal end side of a core shaft made of a superelastic alloy has been subjected to heat treatment.

### CITATION LIST

### Patent Literature

Patent Literature 1: JP 2017-153615 A
Patent Literature 2: JP 2010-222 A
Patent Literature 3: JA H10-146390 A
Patent Literature 4: JP 2005-312987 A

### SUMMARY OF INVENTION

### TECHNICAL PROBLEM

A typical guidewire can sometimes be subjected to "shaping", in which a distal end portion of the guidewire is bent into a predetermined shape so as to conform to the shape of a blood vessel or the like. Guidewires using a core shaft made of a superelastic material have room for improvement in terms of the "shaping performance", which represents the ease of shaping. In particular, the ease of obtaining a "hook shape", in which the guidewire is shaped into a hook shape at an angle of approximately 45 degrees, 1 to 2 millimeters from the distal end, is being sought. In addition, while there is demand to improve the shaping performance, there is also room for improvement in terms of durability of the guidewire with respect to damage, such as folding of the guidewire during use.

The present invention has an object of providing a guidewire having excellent shaping performance and durability, and can be easily shaped into a hook shape.

### SOLUTION TO PROBLEM

The present invention has been made to solve at least a part of the problem described above, and can be realized as the following aspects.

(1) An aspect of the present invention is a guidewire including: a core shaft made of a superelastic material; and a coil formed of a wire that has been spirally wound around an outer periphery of a distal end portion of the core shaft; wherein a section of the core shaft positioned on an inner side of the coil has a first segment consisting of a thermally-transformed portion in which the superelastic material has been thermally transformed, and a second segment that is positioned on a rear end side of the first segment, the second segment is formed having a thermally-transformed portion having a shorter length than the first segment, or is formed not having a thermally-transformed portion, and the coil has a sparsely-wound portion on a distal end portion in which a gap is formed between portions of the wire that are adjacent to each other.

According to such a configuration, the superelastic characteristics of the core shaft are suppressed as a result of providing a first segment consisting of a thermally-transformed portion, and the shaping performance improves. In addition, as a result of a sparsely-wound portion being provided on the distal end portion of the coil, the shaping performance of the distal end portion of the guidewire improves. Consequently, a hook shape can be easily imparted. Furthermore, in the second segment, because a thermally-transformed portion having a shorter length than the thermally-transformed portion on the distal end side is provided, or no thermally-transformed section is provided, the superelasticity of the core shaft is maintained, and the durability of the guidewire can be maintained.

(2) In the guidewire according to the aspect described above, a size of the gap between the portions of the wire in the sparsely-wound portion in a longitudinal direction of the coil may be greater than or equal to an outer diameter of the wire.

According to such a configuration, for example, in a case where a fixing portion that has been formed by a brazing material, a solder material, or the like, is provided in the sparsely-wound portion on the distal end side to fix the core shaft and the coil, the length of the fixed section can be shortened.

(3) **In** the guidewire according to the aspect described above, a rear end of the first segment may be positioned further toward a rear end side than a rear end of the sparsely-wound portion.

**In** a case where the guidewire is subjected to shaping, a "body shape", in which the guidewire is shaped into a gentle curved shape, is imparted in some cases. According to such a configuration, because the first segment consisting of a thermally-transformed portion extends over a wider range, it becomes easier to impart a body shape. Furthermore, because the rear end of the first segment and the rear end of the sparsely-wound portion are provided at different positions in the longitudinal direction, the change in the flexural rigidity of the guidewire can be made more gradual.

(4) **In** the guidewire according to the aspect described above, a rear end of the sparsely-wound portion may be positioned further toward a rear end side than a rear end of the first segment.

According to such a configuration, because the sparsely-wound portion on the distal end side extends over a wider range, it becomes easier to impart a body shape. Furthermore, because the rear end of the sparsely-wound portion and the rear end of the first segment are provided at different positions in the longitudinal direction, the change in the flexural rigidity of the guidewire can be made more gradual.

(5) In the guidewire according to the aspect described above, the coil may further include a sparsely-wound portion on a rear end portion, in which a gap is formed between portions of the wire that are adjacent to each other, and a size of the gap between the portions of the wire in the sparsely-wound portion formed on the rear end portion in a longitudinal direction of the coil may be greater than or equal to an outer diameter of the wire.

According to such a configuration, for example, in a case where a fixing portion that has been formed by a brazing material, a solder material, or the like, is provided in the sparsely-wound portion on the rear end side to fix the core shaft and the coil, the length of the fixed section can be shortened.

Note that the present invention can be implemented in various forms, such as a guidewire, a manufacturing method of a guidewire, a manufacturing method of a catheter, an endoscope, and a dilator.

### BRIEF DESCRIPTION OF DRAWINGS

Fig. 1 is an explanatory diagram illustrating an overall configuration of a guidewire according to a first embodiment.
Fig. 2 is an explanatory diagram illustrating a longitudinal cross-section of a distal end portion of the guidewire.
Fig. 3 is an explanatory diagram illustrating an A-A cross-section of the guidewire.
Fig. 4 is an explanatory diagram illustrating a longitudinal cross-section of a distalmost end of the guidewire.
Fig. 5 is an explanatory diagram illustrating a longitudinal cross-section of a rear end portion of a coil.
Fig. 6 is an explanatory diagram illustrating a distal end portion of a guidewire after shaping.
Fig. 7 is an explanatory diagram illustrating a longitudinal cross-section of a distal end portion of a guidewire according to a second embodiment.
Fig. 8 is an explanatory diagram illustrating a longitudinal cross-section of a distal end portion of a guidewire according to a third embodiment.
Fig. 9 is an explanatory diagram illustrating a longitudinal cross-section of a distal end portion of a guidewire according to a fourth embodiment.
Fig. 10 is an explanatory diagram illustrating a distal end portion of a guidewire after being shaped into a hook shape.
Fig. 11 is an explanatory diagram illustrating a guidewire according to a first modification.
Fig. 12 is an explanatory diagram illustrating a guidewire according to a fourth modification.
Fig. 13 is an explanatory diagram illustrating a guidewire according to an eighth modification.

### EMBODIMENTS OF THE INVENTION

### <First Embodiment>

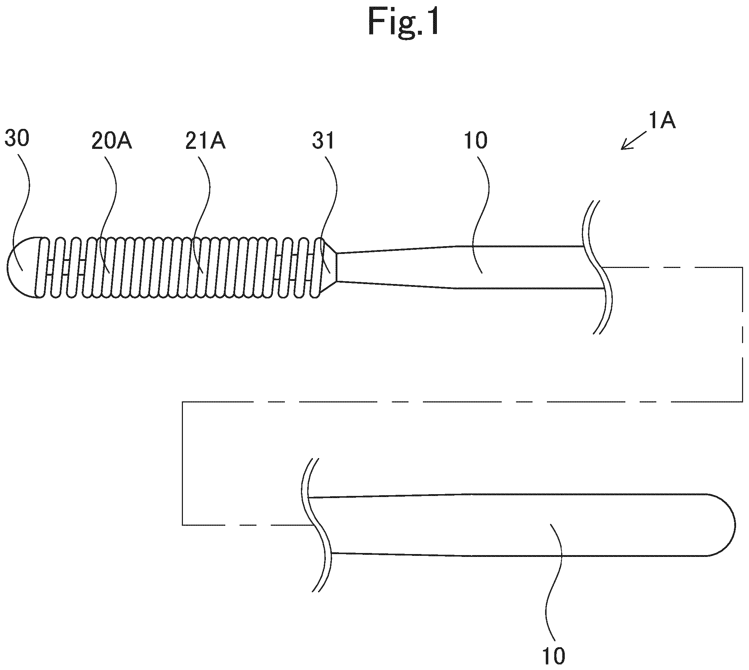

The size of each constituent member of the guidewire 1A according to the first embodiment shown in Figs. 1 to 6 are illustrations, and may be shown in a different scale to the actual scale in some cases. In the following, the end portion located on the distal end side of each constituent member of the guidewire 1A is referred to as the "distal end", and a part including the "distal end" that extends partway from the distal end toward the rear end side is referred to as the "distal end portion". Similarly, the end portion located on the rear end side of each constituent member is referred to as the "rear end", and a part including the "rear end" that extends partway from the rear end toward the distal end side is referred to as the "rear end portion".

Fig. 1 is an explanatory diagram illustrating an overall view of the guidewire 1A according to the first embodiment. The guidewire 1A is a medical device used for the treatment of blood vessels and the like. The guidewire 1A includes a core shaft 10, a coil 20A, a distal end fixing portion 30, and a rear end fixing portion 31.

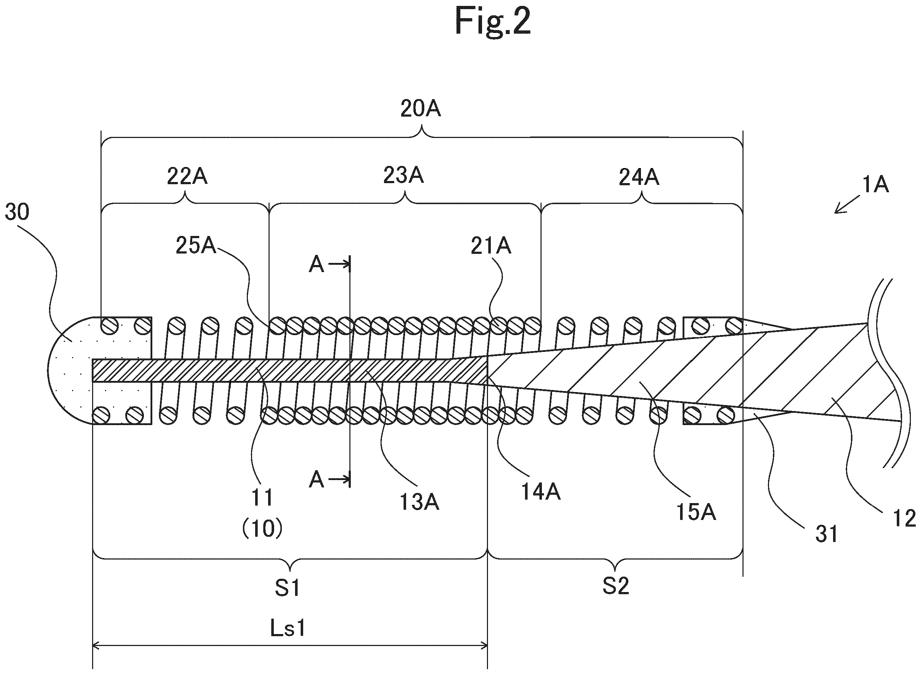

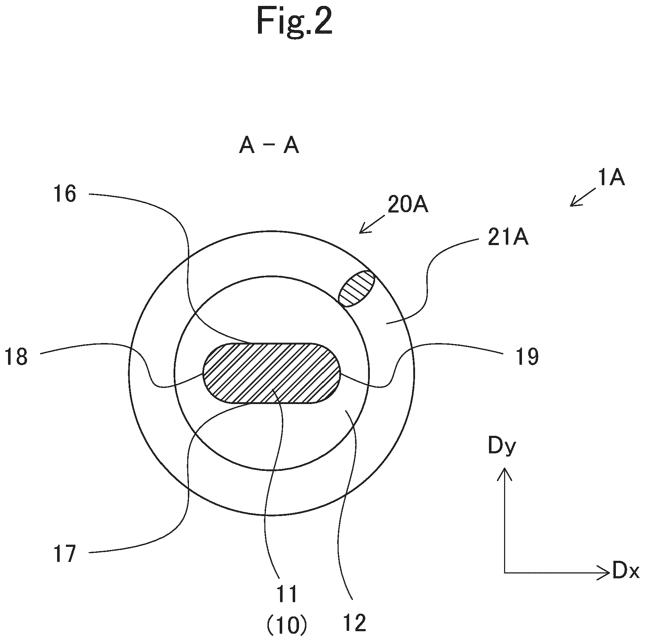

Fig. 2 is an explanatory diagram illustrating a longitudinal cross-section of a distal end portion of the guidewire 1A according to the first embodiment. The core shaft 10 is a long member that extends from the distal end of the guidewire 1A to the rear end. The core shaft 10 is formed such that the outer diameter becomes smaller toward the distal end side, and the distal end portion includes a straight portion 11 whose outer diameter is substantially constant in the longitudinal direction, and a tapered portion 12 provided on the rear end side of the straight portion 11. The core shaft 10 will be described later in more detail.

The core shaft 10 is formed of a superelastic material. The superelastic material is not particularly limited, but examples of superelastic alloys that can be used include Ni-Ti alloys, Ni-Ti-X alloys (where X = Fe, Cu, V, Co, Cr, Mn, Nb, and the like), and Cu-Zn-X alloys (where X = Al, Fe, and the like).

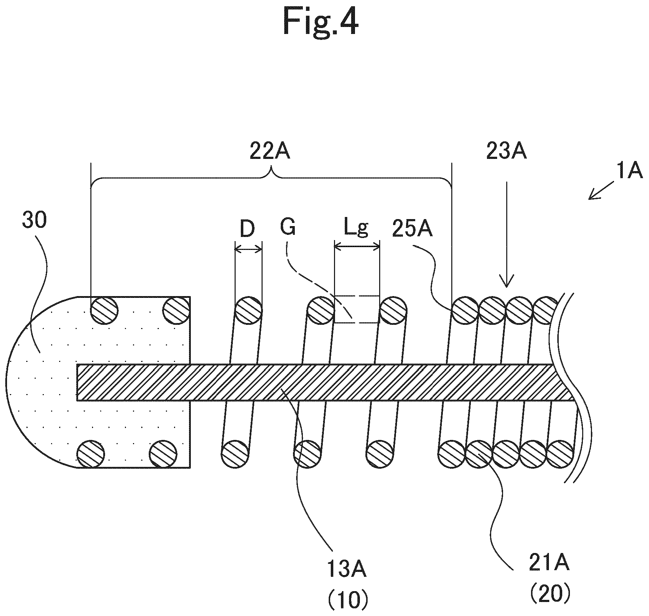

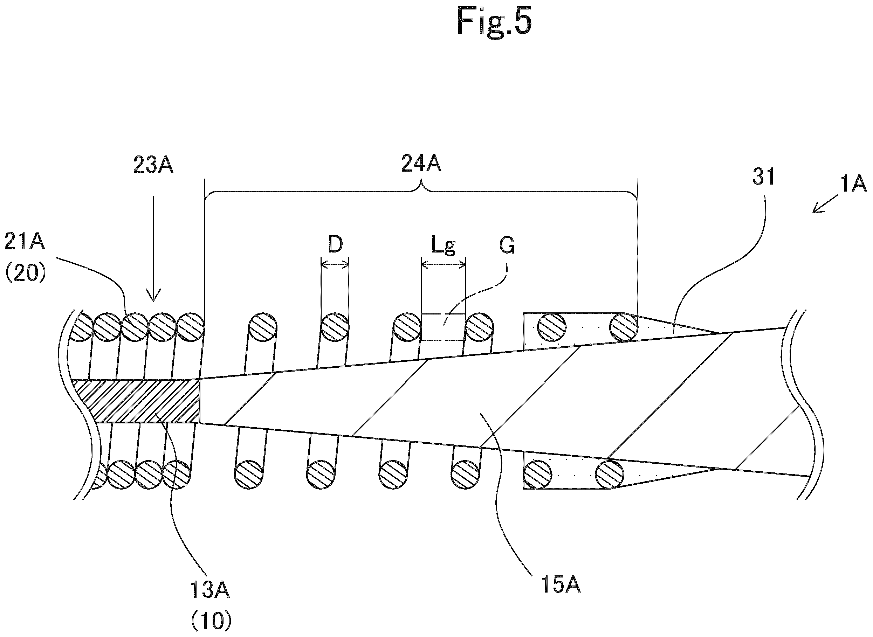

The coil 20A is a member formed by a wire 21A that has been wound in a spiral shape so as to cover the outer periphery of the distal end portion of the core shaft 10. The coil 20A includes, in order from the distal end side, a sparsely-wound portion 22A, a densely-wound portion 23A, and a sparsely-wound portion 24A. The densely-wound portion 23A is a section having a small pitch in which the wire 21A is wound so as to make contact with itself. On the other hand, the sparsely-wound portion 22A and the sparsely-wound portion 24A have a larger pitch than the densely-wound portion 23A, and are sections in which gaps G described below are formed between the portions of the wire 21A that are adjacent to each other (see Figs. 4 and 5). **In** the present embodiment, although the coil 20A has the densely-wound portion 23A and the sparsely-wound portion 24A further toward the rear end side than the sparsely-wound portion 22A, the entire length of the coil 20A may be configured by the sparsely-wound portion 22A. Furthermore, the entire section of the coil 20A further toward the rear end side than the sparsely-wound portion 22A may be the densely-wound portion 23A. The configuration of the sparsely-wound portion 22A of the coil 20A is not limited to the example described above, and various modifications are possible. The details of the sparsely-wound portion 22A and the sparsely-wound portion 24A will be described later.

The material of the coil 20A is not particularly limited, but examples of materials that can be used include stainless steel (such as SUS302, SUS304, and SUS316), superelastic alloys such as Ni-Ti alloys, piano wire, nickel-chromium alloys, cobalt alloys, tungsten, and platinum.

The distal end fixing portion 30 is a member that fixes the distal end of the core shaft 10 and the distal end of the coil 20A. Furthermore, the rear end fixing portion 31 is a member that fixes an intermediate section of the core shaft 10 and the rear end of the coil 20A. For example, the distal end fixing portion 30 and the rear end fixing portion 31 are formed by applying a brazing material or solder material in a molten state to the core shaft 10 and the coil 20A, and then cooling and solidifying the material.

The material of the distal end fixing portion 30 and the rear end fixing portion 31 is not particularly limited, but examples of materials that can be used include brazing materials (such as aluminum alloy brazing, silver brazing, and gold brazing), metal solder (such as Ag-Sn alloys and Au-Sn alloys), and adhesives (such as epoxy-based adhesives).

### <Details of Core Shaft 10>

The details of the core shaft 10 will now be described. The core shaft 10 includes, on the inner side of the coil 20A, a first segment S1 and a second segment S2. The first segment extends from the distal end of the core shaft 10 toward the rear end side, and consists of a thermally-transformed portion 13A. Here, the section of the core shaft 10 excluding the thermally-transformed portion 13A that has not been thermally transformed is referred to as a "non-thermally-transformed portion 15A". The thermally-transformed portion 13A is a section in which the characteristics of the superelastic material forming the core shaft 10 have been changed by heating the core shaft 10. In the present embodiment, the thermally-transformed portion 13A is a section in which the superelastic characteristics of the core shaft 10 are more suppressed than in the non-thermally-transformed portion 15A. Furthermore, a rear end 14A of the first segment S1 (rear end of the thermally-transformed portion 13A) represents the boundary between the thermally-transformed portion 13A and the non-thermally-transformed portion 15A. The rear end 14A of the first segment S1 is disposed further toward the rear end side than a rear end 25A of the sparsely-wound portion 22A described below. As a result, the rear end 14A of the first segment S1 and the rear end 25A of the sparsely-wound portion 22A on the distal end side are provided at different positions in the longitudinal direction of the core shaft 10. Fig. 2 indicates the length from the distal end to the rear end 14A of the first segment S1 in the longitudinal direction as Ls1. The length Ls1 of the first segment is not particularly limited, but may be, for example, 2 millimeters to 7 millimeters.

The second segment S2 is on the inner side of the coil 20A, and is positioned further toward the rear end side than the first segment S1. The second segment S2 is formed without the thermally-transformed portion 13A, and is formed of only the non-thermally-transformed portion 15A. In the present embodiment, although the thermally-transformed portion 13A is not formed in the second segment S2, the thermally-transformed portion 13A may be formed in the second segment S2 as described in a second embodiment below. In this case, the length in the longitudinal direction of the thermally-transformed portion 13A that is formed in the second segment S2 is shorter than the length of the thermally-transformed portion 13A in the first segment S1.

Although the method of producing the thermally-transformed portion 13A is not particularly limited, for example, the thermally-transformed portion 13A can be formed by irradiating the surface of the core shaft 10 with a laser, and heating the core shaft 10 to a temperature of approximately 600 degrees to 1,000 degrees.

Fig. 3 is an explanatory diagram illustrating an A-A cross-section of the guidewire 1A according to the first embodiment. The straight portion 11 of the core shaft 10 is formed of a flat surface 16, a flat surface 17 opposing the flat surface 16, and a curved surface 18 and curved surface 19 that connect the flat surface 16 and the flat surface 17. The transverse cross-section of the straight portion 11 has a flattened shape that extends long in a predetermined direction. In the present embodiment, the length of the transverse cross-section of the straight portion 11 in a direction Dx that is parallel to the flat surface 16 is longer than the length in a direction Dy that is perpendicular to the flat surface 16. As a result, the ease of bending of the straight portion 11 differs depending on the direction of the stress applied to the straight portion 11. For example, the straight portion 11 is more easily bent in a case where stress is applied to the straight portion 11 in the direction Dy that is perpendicular to the flat surface 16 than in a case where stress is applied to the direction Dx that is parallel to the flat surface 16. Here, although the length of the straight portion 11 in the longitudinal direction of the guidewire 1A is not particularly limited, for example, the length can be 10 to 20 millimeters. Furthermore, although not illustrated, the transverse cross-section of the tapered portion 12, and the core shaft 10 further toward the rear end side than the tapered portion 12, has a circular shape.

Although the method of producing the straight portion 11 is not particularly limited, for example, the straight portion 11 can be produced by subjecting the distal end portion of the core shaft 10 formed in a cylindrical shape to press treatment using a die provided with a flat surface portion.

### <Details of Sparsely-Wound Portion 22A on Distal End Side>

Fig. 4 is an explanatory diagram illustrating a longitudinal cross-section of the distalmost end of the guidewire 1A according to the first embodiment. The details of the sparsely-wound portion 22A will now be described. The coil 20A has the sparsely-wound portion 22A, in which, of the wire 21A that has been spirally wound, the gaps G are provided between the portions of the wire 21A that are adjacent to each other. The sparsely-wound portion 22A is formed from the distal end of the coil 20A toward the rear end side, and the distal end is embedded in the distal end fixing portion 30. The rear end 25A of the sparsely-wound portion 22A represents the boundary between the sparsely-wound portion 22A and the densely-wound portion 23A. Although the length of the sparsely-wound portion 22A in the longitudinal direction is not limited, for example, the length can be 0.5 millimeters to 2 millimeters.

When the outer diameter of the wire 21A that constitutes the sparsely-wound portion 22A is D, and the size of the gaps G between portions of the wire 21A in the longitudinal direction is Lg, the size Lg of the gaps G between the portions of the wire 21A is greater than or equal to the outer diameter D of the wire 21A. Here, the outer diameter D of the wire 21A is the size of the wire 21A in the longitudinal direction of the coil 20A. Furthermore, in a case where, as in the wire 21A of the present embodiment, the wire 21A has a circular shape, and the size of the wire 21A in the longitudinal direction of the coil 20A is not constant in a transverse cross-section, the outer diameter D of the wire 21A is defined as the "size of the wire 21A in the longitudinal direction at the section of the coil 20A in which the size in the longitudinal direction is the largest". That is, the maximum outer diameter of the wire 21A is used as the outer diameter D. Here, a configuration in which the size Lg of the gaps G and the outer diameter D of the wire 21A are substantially constant can also be stated as being a configuration in which, when the size of the pitch of the densely-wound portion 23A that is wound such that the wire 21A makes contact with itself is 100%, the size of the pitch of the sparsely-wound portion 22A is approximately 200%. Therefore, "the size Lg of the gaps G is greater than or equal to the outer diameter D of the wire 21A" can also be stated as the size of the pitch of the sparsely-wound portion 22A being 200% or more when the size of the pitch of the densely-wound portion 23A is 100%. In a case where, as in the sparsely-wound portion 22A shown in Fig. 4, the gaps G are formed between the portions of the wire 21A that are adjacent to each other, the brazing material or solder material of the distal end fixing portion 30 is fixed in a state where the material has flowed into the gaps G in the sparsely-wound portion 22A.

### <Details of Sparsely-Wound Portion 24A on Rear End Side>

Fig. 5 is an explanatory diagram illustrating a longitudinal cross-section of the rear end portion of the coil 20A of the guidewire 1A according to the first embodiment. The details of the sparsely-wound portion 24A will now be described. The rear end portion of the coil 20A is formed having the sparsely-wound portion 24A. The sparsely-wound portion 24A is formed from the rear end of the coil 20A toward the distal end side, and the rear end is embedded in the rear end fixing portion 31. The sparsely-wound portion 24A formed on the rear end side, like the sparsely-wound portion 22A formed on the distal end side described above, has the gaps G formed between the portions of the wire 21A that are adjacent to each other. Although the length of the sparsely-wound portion 24A in the longitudinal direction is not limited, for example, the length can be 0.5 millimeters to 2 millimeters. When the size of the pitch of the densely-wound portion 23A is 100%, the size of the pitch of the sparsely-wound portion 24A shown in Fig. 5 is 200% or more.

In the present embodiment, although the size Lg of the gaps G in the sparsely-wound portion 22A and the sparsely-wound portion 24A is greater than or equal to the outer diameter D of the wire 21A, the size Lg of the gaps G in the sparsely-wound portion 22A and the sparsely-wound portion 24A may also be smaller than the outer diameter D of the wire 21A. When the pitch of the densely-wound portion 23A is 100% as mentioned above, the size of the pitch of the sparsely-wound portion 22A and the sparsely-wound portion 24A can be approximately 150% to 500%. As a result of the size of the pitch of the sparsely-wound portion 22A and the sparsely-wound portion 24A being approximately 150% or more, the portions of the wire 21A that are adjacent to each other are sufficiently separated, and the thermal conductivity between the portions of the wire 21A decreases. As a result, in a case where a brazing material or a solder material is used for the distal end fixing portion 30, the amount of molten brazen material or solder material that flows in the longitudinal direction along the wire 21A can be reduced.

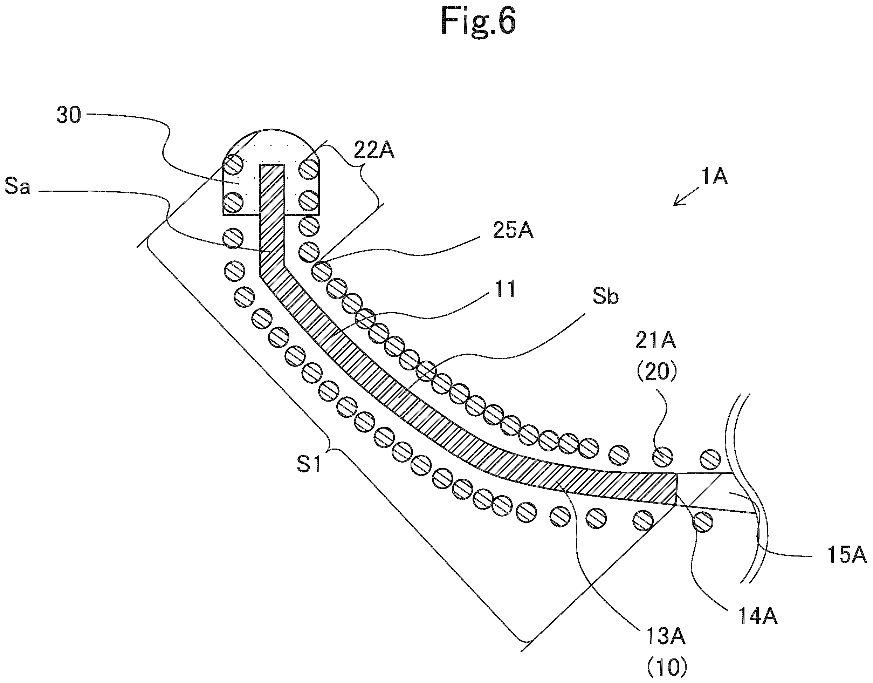

Fig. 6 is an explanatory diagram illustrating the distal end portion of the guidewire 1A after shaping. As mentioned above, in the present specification, shaping of the guidewire 1A approximately 1 to 2 millimeters from the distal end into a hook shape at an angle of approximately 45 degrees is referred to as a "hook shape", and imparting a gentle curve shape approximately 20 millimeters from the distal end of the guidewire 1A is referred to as a "body shape". In Fig. 6, the section in which the hook shape has been imparted is indicated by a hook shape portion Sa, and the section in which the body shape has been imparted is indicated by a body shape portion Sb. The hook shape portion Sa is a section in which the thermally-transformed portion 13A and the sparsely-wound portion 22A are provided overlapping each other. In addition, the thermally-transformed portion 13A is also provided in the body shape portion Sb. Further, because the transverse cross-section of the straight portion 11 has a flattened shape, the straight portion 11 has a direction in which it is more easily bent. In this way, the guidewire 1A is provided with a section at the distalmost end that is most easily shaped, and is provided with a section further toward the rear end side than the distalmost end that is also easily shaped. That is, the ease of shaping of the guidewire 1A changes in steps from the distal end side toward the rear end side.

According to the guidewire 1A of the present embodiment described above, the guidewire 1A includes the first segment S1 consisting of the thermally-transformed portion 13A. Because the thermally-transformed portion 13A has superelastic characteristics that are suppressed compared to the non-thermally-transformed portion 15A, the force that acts to try to return to the original shape when deformation occurs is reduced. As a result, the guidewire 1A can be more easily shaped in an intended direction.

The guidewire 1A has the sparsely-wound portion 22A, and the size Lg of the gaps G between the portions of the wire 21A that are adjacent to each other is greater than or equal to the outer diameter D of the wire 21A. That is, while the pitch of the coil of a typical guidewire is approximately 100% to 120%, the pitch in the sparsely-wound portion 22A is widened to 200% or more. As a result of the portions of the wire 21A that are adjacent to each other in the sparsely-wound portion 22A being sufficiently separated, the thermal conductivity between the portions of the wire 21A decreases. As a result, in a case where a brazing material or a solder material is used for the distal end fixing portion 30, the amount of molten brazen material or solder material that flows in the longitudinal direction along the wire 21A can be reduced. Therefore, the length of the distal end fixing portion 30 in the longitudinal direction can be made shorter, and the flexibility of the distal end of the guidewire 1A can be improved. As a result of sparsely winding the coil 20A to make the coil 20A itself flexible, and shortening the length of the distal end fixing portion 30, the distal end portion of the guidewire 1A can be made flexible, and a user can more easily shape the guidewire 1A in an intended direction. Specifically, if the length of the sparsely-wound portion 22A in the longitudinal direction is made approximately 1 to 2 millimeters, it becomes easier to impart an approximately 1 to 2 millimeter hook shape. As a result of imparting a hook shape, even in a case where the guidewire 1A is inserted into a branched portion of a blood vessel having a small blood vessel diameter (for example, a blood vessel having a diameter of approximately 1 to 2 millimeters), a user can easily advance the guidewire 1A in an intended direction.

The rear end 14A of the first segment S1 is disposed further toward the rear end side than the rear end 25A of the sparsely-wound portion 22A. Consequently, the section of the core shaft 10 that can be easily shaped is more widely disposed, which makes it easier to impart a body shape. For example, as a result of the length Ls1 of the first segment S1 in the longitudinal direction (Fig. 2) being approximately 20 millimeters, it becomes easier to impart a body shape of approximately 20 millimeters. As a result of imparting a body shape, even in a case where the guidewire 1A is inserted into a branched portion of a blood vessel having a large blood vessel diameter (for example, a blood vessel having a diameter of approximately 2 millimeters to 10 millimeters), a user can easily advance the guidewire 1A in an intended direction. Furthermore, even when the length of the first segment S1 is 2 to 7 millimeters, it becomes easier to impart a body shape because the thermally-transformed portion 13A is provided at the distal end portion which, of the body shape portion Sb, has a large amount of deformation when shaping is performed. When the length Ls1 of the first segment S1 is shortened, the length of the second segment S2 becomes relatively long, and it becomes easier to maintain both the shaping performance and durability because the durability with respect to bending and folding of the core shaft 10 improves.

As a result of the rear end 14A of the first segment S1 being disposed further toward the rear end side than the rear end 25A of the sparsely-wound portion 22A, the rear end 14A of the first segment S1 and the rear end 25A of the sparsely-wound portion 22A on the distal end side are provided at different positions in the longitudinal direction of the core shaft 10. Consequently, the change in the flexural rigidity of the core shaft 10 at the boundary between the thermally-transformed portion 13A and the non-thermally-transformed portion 15A, and the change in the flexural rigidity of the coil 20A at the boundary between the sparsely-wound portion 22A and the densely-wound portion 23A (the rear end 15A of the sparsely-wound portion 22A) occur at different positions. Therefore, sudden changes in the flexural rigidity in the longitudinal direction of the guidewire 1A can be suppressed.

The thermally-transformed portion 13A is not provided in the second segment S2, and the second segment S2 is formed of only the non-thermally-transformed portion 15A. As a result, the superelastic characteristics of the core shaft 10 are maintained in the second segment S2, and unintended folding and bending of the guidewire 1A can be suppressed. As a result of the above, the shaping performance of the guidewire 1A improves, and further, the durability of the guidewire 1A can be improved.

The coil 20A has the sparsely-wound portion 24A on the rear end side, and the size Lg of the gaps G between the portions of the wire 21A that are adjacent to each other is greater than or equal to the outer diameter D of the wire 21A. **In** contrast to the pitch of the coil of a typical guidewire, which is approximately 100% to 120%, the pitch in the sparsely-wound portion 22A is widened to 200% or more. As a result, like the sparsely-wound portion 22A described above, because the portions of the wire 21A that are adjacent to each other in the sparsely-wound portion 24A are sufficiently separated, the thermal conductivity between the portions of the wire 21A decreases. Consequently, in a case where a brazing material or a solder material is used for the rear end fixing portion 31, the amount of molten brazen material or solder material that flows in the longitudinal direction along the gaps G of the wire 21A can be reduced. Therefore, the length of the rear end fixing portion 31 in the longitudinal direction can be made shorter, and the flexibility of the guidewire 1A can be improved. As a result, the occurrence of an increase in the flexural rigidity near the rear end fixing portion 31 of the guidewire 1A can be suppressed, and the possibility of damage occurring such as folding or bending of the guidewire 1A near the rear end fixing portion 31 can be reduced.

The transverse cross-section of the straight portion 11 of the core shaft 10 has a flattened shape that extends long in a predetermined direction. As a result, the direction in which the straight portion 11 can be easily bent differs depending on the direction of the stress applied to the straight portion 11. In a case where the core shaft 10 is to be shaped, because the straight portion 11 tries to bend in the easily bent direction, the guidewire 1A can be uniformly bent in a fixed direction. In the present embodiment, shaping can be easily performed by uniformly bending the distal end portion of the guidewire in the Dy direction shown in Fig. 3. For example, in a case where the transverse cross-section of the straight portion 11 is not a flattened shape, and shape of the transverse cross-section is a circular shape, because the ease of bending does not depend on the direction and is constant, it is difficult to uniformly bend the guidewire 1A in a fixed direction, and the possibility of the guidewire 1A being shaped so as to be twisted increases.

### <Second Embodiment>

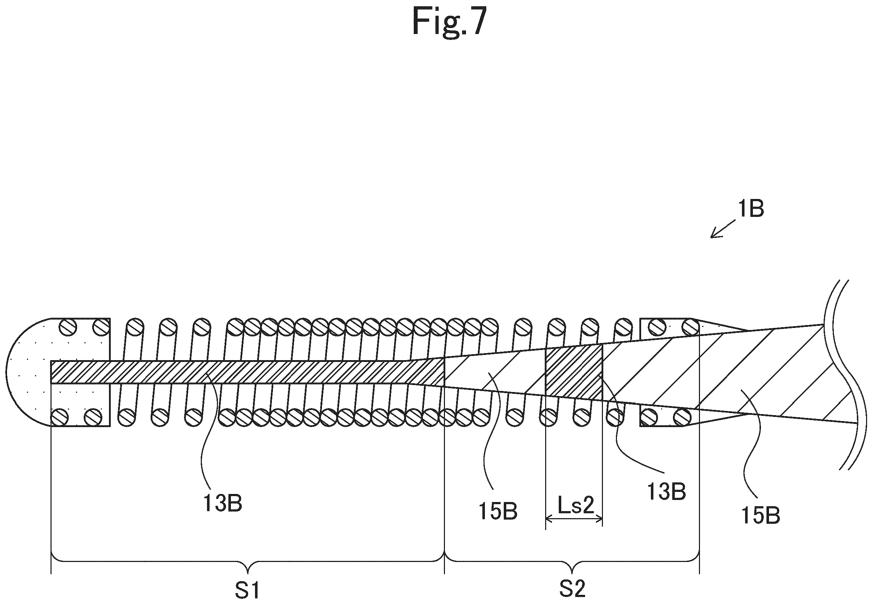

Fig. 7 is an explanatory diagram illustrating a longitudinal cross-section of the distal end portion of a guidewire 1B according to a second embodiment. The guidewire 1B is different from the guidewire 1A according to the first embodiment in that the thermally-transformed portion 13B is provided in the second segment S2. The description of the configurations of the guidewire 1B that are common to the guidewire 1A will be omitted.

A portion of the second segment S2 is provided with the thermally-transformed portion 13B, and the non-thermally-transformed portion 15B is provided in the sections other than the thermally-transformed portion 13B. The length Ls2 in the longitudinal direction of the thermally-transformed portion 13B that is provided in the second segment S2 is shorter than the length Ls 1 of the first segment S1 in the longitudinal direction.

In a configuration in which the thermally-transformed portion 13B is provided in the second segment S2 as in the guidewire 1B, because the length Ls2 in the longitudinal direction of the thermally-transformed portion 13B that is provided in the second segment S2 is shorter than the length Ls1 of the first segment S1 in the longitudinal direction, the durability with respect to folding and bending of the core shaft 10 can be improved.

### <Third Embodiment>

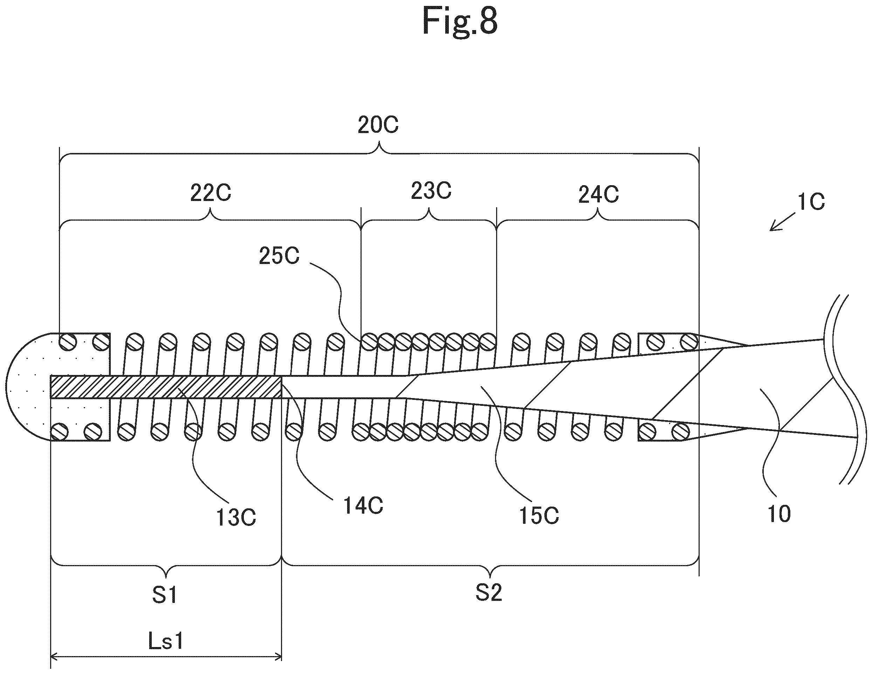

Fig. 8 is an explanatory diagram illustrating a longitudinal cross-section of the distal end portion of a guidewire 1B according to a third embodiment. The guidewire 1C is different from the guidewire 1A according to the first embodiment in that the rear end 25C of the sparsely-wound portion 22C is positioned further toward the rear end side than the rear end 14C of the first segment S1. The description of the configurations of the guidewire 1C that are common to the guidewire 1A will be omitted.

Because the guidewire 1C has the sparsely-wound portion 22C extending further toward the rear end side and provided over a wider area, it becomes easier to impart a body shape. Furthermore, in the present embodiment, the rear end 14C of the first segment S1 and the rear end 25C of the sparsely-wound portion 22C on the distal end side are provided at different positions in the longitudinal direction of the core shaft 10. As a result, it is possible to suppress a sudden change in the flexural rigidity caused by an overlap between the position at which a change in the flexural rigidity of the core shaft 10 occurs and the position at which a change in the flexural rigidity of the coil 20C occurs.

### <Fourth Embodiment>

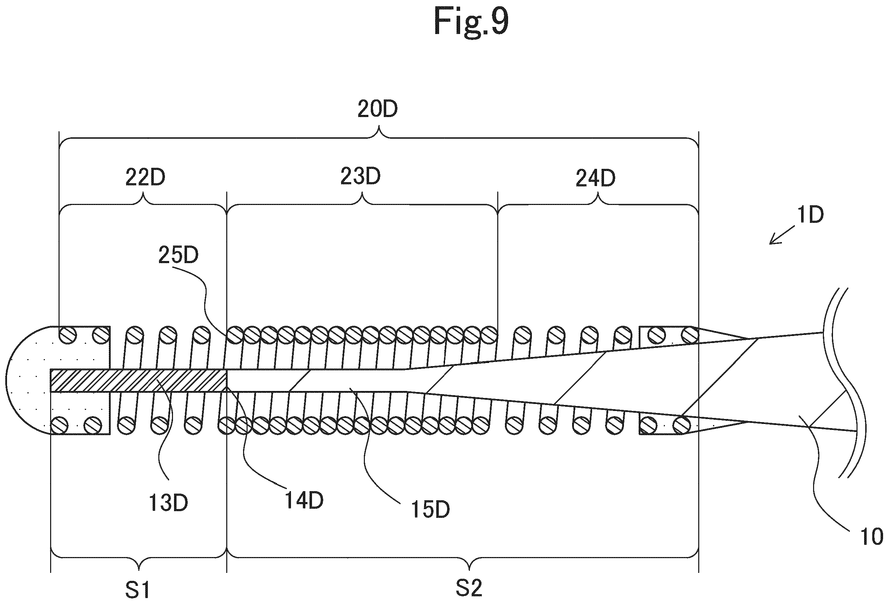

Fig. 9 is an explanatory diagram illustrating a longitudinal cross-section of the distal end portion of a guidewire 1D according to a fourth embodiment. The guidewire 1D differs from the guidewire 1A according to the fourth embodiment in that the rear end 14D of the first segment S1 and the rear end 25D of the sparsely-wound portion 22D are at substantially the same position in the longitudinal direction of the coil 20C. The description of the configurations of the guidewire 1D that are common to the guidewire 1A will be omitted.

In the guidewire 1D, a hook shape can be easily imparted due to the thermally-transformed portion 13D and the sparsely-wound portion 22A. Furthermore, even in a mode such as in the present embodiment where the thermally-transformed portion 13D does not extend further toward the rear end side than the rear end 25D of the sparsely-wound portion 22D, because the core shaft 10 has the straight portion 11 having a flattened shape in a transverse cross-section, it becomes easier to impart a body shape. In addition, by setting the thermally-transformed portion 13D to be short, the range in which the durability of the core shaft 10 is maintained can be more widely ensured.

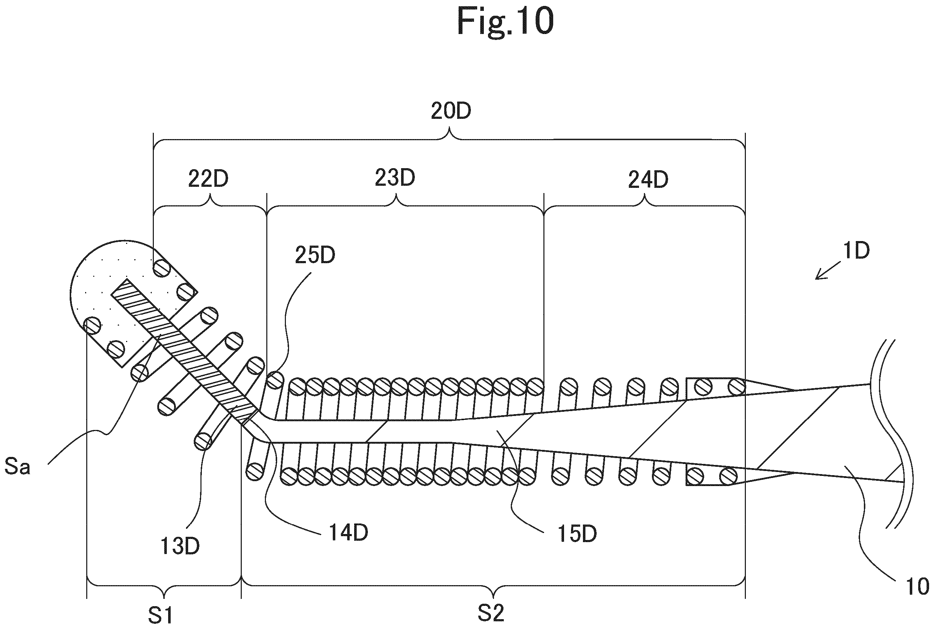

Fig. 10 is an explanatory diagram illustrating the distal end portion of the guidewire 1D according to the fourth embodiment after being shaped into a hook shape. In Fig. 10, the section in which the hook shape has been imparted is indicated by the hook shape portion Sa. Of the guidewire 1D described above, because the section in which the thermally-transformed portion 13D and the sparsely-wound portion 22D is provided can be easily shaped, it becomes particularly easy to impart a hook shape.

### <Modifications>

The present invention is not limited to the embodiments described above, and may be implemented in various modes without departing from the gist thereof, and for example, the following modifications are also possible.

### <First Modification>

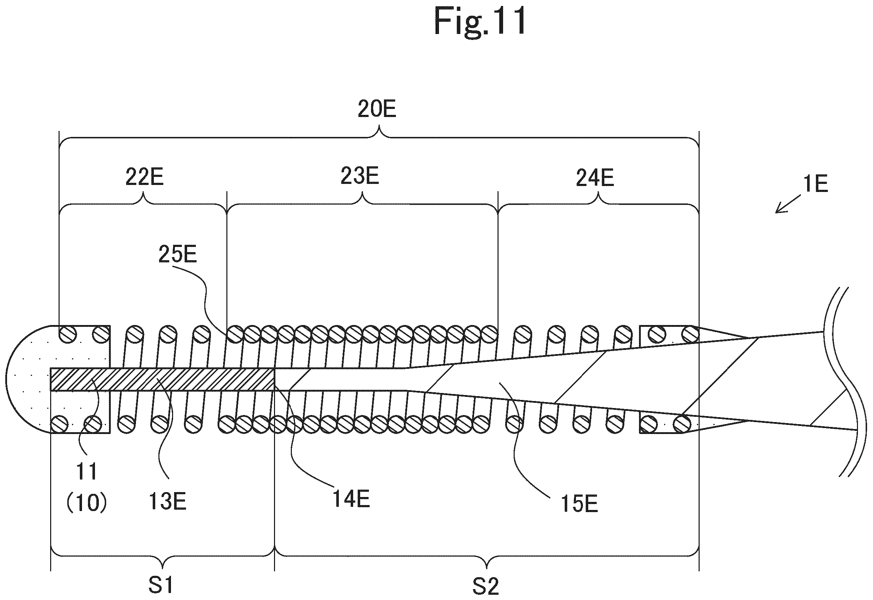

Fig. 11 is an explanatory diagram illustrating a guidewire 1E according to a first modification. In the guidewire 1E, the position of the rear end 14E of the first segment S1 is different from that of the guidewires according to the embodiments. In the guidewire 1A according to the first embodiment, the rear end 14A of the first segment S1 is further toward the rear end side than the rear end 25E of the sparsely-wound portion 22A and is formed over the entire length of the straight portion 11. However, the rear end 14E of the first segment S1 may, as in the guidewire 1E, be provided further toward the rear end side than the rear end 25E of the sparsely-wound portion 22E, and in a middle portion of the straight portion 11. In the guidewire 1E, a hook shape can more be easily imparted due to the thermally-transformed portion 13E and the sparsely-wound portion 22E. Furthermore, the straight portion 11 enables a body shape to be uniformly imparted in a predetermined direction. Also, by setting the thermally-transformed portion 13E to be short, the durability of the core shaft 10 can be maintained over a wide range.

### <Second Modification>

In the guidewires according to the first to fourth embodiments (1A, 1B, 1C, 1D), although the first segment S1 is formed from the distalmost end of the core shaft 10 toward the rear end side, the first segment S1 does not have to be formed from the distalmost end of the core shaft 10. The first segment S1 only needs to be formed on the inner side of the coil (20A, 20B, 20C, 20D), and for example, the distal end of the first segment S1 may be provided further toward the rear end side than the distal end of the core shaft 10.

### <Third Modification>

The first segment S1 and the second segment S2 may be provided with a section other than the thermally-transformed portion (13A, 13B, 13C, 13D) in which the characteristics of the core shaft 10 are changed. For example, a section may be provided in which the surface state of the core shaft 10 is changed by electrolytic polishing, shot peening, and the like.

### <Fourth Modification>

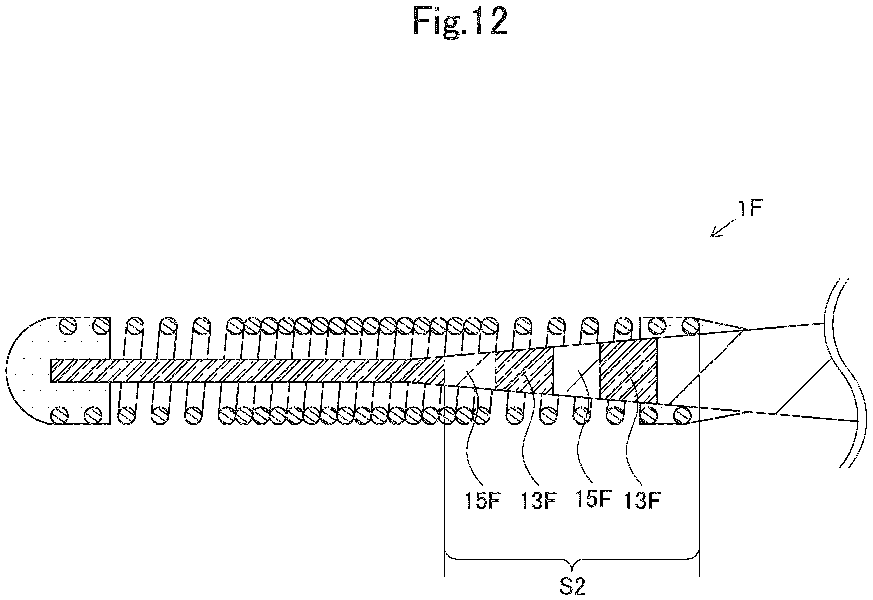

Fig. 12 is an explanatory diagram illustrating a guidewire 1F according to a fourth modification. The guidewire 1F has a plurality of thermally-transformed portions 13F in the second segment. Two thermally-transformed portions 13F may be provided in the second segment S2 as in the guidewire 1F.

### <Fifth Embodiment>

In the guidewires according to the first to fourth embodiments (1A, 1B, 1C, 1D), although the section of the core shaft 10 positioned further toward the rear end side than the second segment S2 is not provided with the thermally-transformed portion (13A, 13B, 13C, 13D), the thermally-transformed portion (13A, 13B, 13C, 13D) may also be provided.

### <Sixth Embodiment>

In the guidewires according to the first to fourth embodiments (1A, 1B, 1C, 1D), although the transverse cross-section of the wire (21A, 21B, 21C, 21D) has a circular shape, a tetragonal shape such as a square shape or a rectangular shape is also possible.

### <Seventh Modification>

In the guidewires according to the first to fourth embodiments (1A, 1B, 1C, 1D), although the coil (20A, 20B, 20C, 20D) is formed using a single wire (21A, 21B, 21C, 21D), the coil may be formed by winding a plurality of wires (21A, 21B, 21C, 21D) in a spiral shape. In this case, the outer diameter D of the wire (21A, 21B, 21C, 21D) refers to the outer diameter D among the plurality of wires (21A, 21B, 21C, 21D) having the largest outer diameter D. In addition, the plurality of wires (21A, 21B, 21C, 21D) may be bundled together to form a strand, and the strand may be wound in a spiral shape to form the coil (20A, 20B, 20C, 20D). In this case, the gaps G represent the gaps between portions of the strand that are adjacent to each other, and the outer diameter of the strand is the outer diameter D of the wire (21A, 21B, 21C, 21D).

### <Eighth Modification>

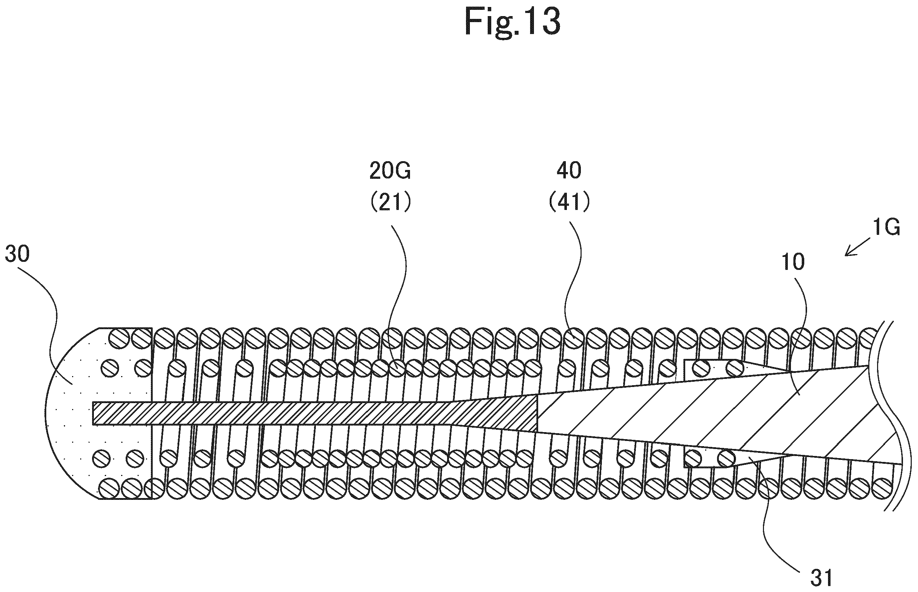

Fig. 13 is an explanatory diagram illustrating a guidewire 1G according to an eighth modification. The guidewire 1G has a plurality of coils (20G, 40). In the guidewires according to the first to fourth embodiments (1A, 1B, 1C, 1D), although the outer periphery of the core shaft 10 is only covered by a single coil (20A, 20B, 20C, 20D), a separate coil 40 may be provided on the outer periphery of the coil (20A, 20B, 20C, 20D). The guidewire 1G illustrated in Fig. 13 has the coil 40 further covering the outer periphery of the coil 20G, which covers the outer periphery of the core shaft 10. The coil 40 is formed as a result of a wire 41 that is separate to the wire 21G constituting the coil 20G being wound in a spiral shape.

### <Ninth Modification>

In the guidewire 1A according to the first embodiment, although the straight portion 11 has a flattened shape, and is formed of the flat surface 16, the flat surface 17 opposing the flat surface 16, and the curved surface 18 and the curved surface 19 that connect the flat surface 16 and the flat surface 17, the straight portion 11 does not have to be formed of the flat surface 16, the flat surface 17, the curved surface 18, and the curved surface 19. For example, the straight portion 11 may be formed of four flat surfaces, and in this case, the straight portion 11 can be formed having a flattened shape by adjusting arbitrary widths of the flat surfaces. In addition, the straight portion 11 does not have to have a flattened shape, and for example, a tetragonal shape in a transverse cross-section, such as square shape or a rectangular shape, or a circular shape are also possible. Furthermore, the tapered portion 12 and the section of the core shaft 10 on the rear end side may also have a flattened shape.

### DESCRIPTION OF REFERENCE NUMERALS

1A Guidewire
10 Core shaft
11 Straight portion
12 Tapered portion
13A Thermally-transformed portion
14A Rear end of thermally-transformed portion
15A Non-thermally-transformed portion
16 Flat surface
17 Flat surface
18 Curved surface
19 Curved surface
20A Coil
21A Wire
22A Sparsely-wound portion
23A Densely-wound portion
24A Sparsely-wound portion
25A Rear end of sparsely-wound portion
30 Distal end fixing portion
31 Rear end fixing portion
Ls1 Length of first segment
Ls2 Length of thermally-transformed portion of second segment
D Outer diameter of wire
G Gap between wire
Lg Length of gap
Sa Hook shape portion
Sb Body shape portion

## Claims

1. A guidewire comprising:
a core shaft made of a superelastic material; and
a coil formed of a wire that has been spirally wound around an outer periphery of a distal end portion of the core shaft; wherein
a section of the core shaft positioned on an inner side of the coil has a first segment consisting of a thermally-transformed portion in which the superelastic material has been thermally transformed, and a second segment that is positioned on a rear end side of the first segment,
the second segment is formed having the thermally-transformed portion having a shorter length than the first segment, or is formed not having the thermally-transformed portion, and
the coil has a sparsely-wound portion on a distal end portion in which a gap is formed between portions of the wire that are adjacent to each other.

2. The guidewire according to claim 1, wherein
a size of the gap between the portions of the wire in the sparsely-wound portion in a longitudinal direction of the coil is greater than or equal to an outer diameter of the wire.

3. The guidewire according to claim 1 or 2, wherein
a rear end of the first segment is positioned further toward a rear end side than a rear end of the sparsely-wound portion.

4. The guidewire according to claim 1 or 2, wherein
a rear end of the sparsely-wound portion is positioned further toward a rear end side than a rear end of the first segment.

5. The guidewire according to any one of claims 1 to 4, wherein
the coil is further provided with a sparsely-wound portion on a rear end portion, in which a gap is formed between portions of the wire that are adjacent to each other, and
a size of the gap between the portions of the wire in the sparsely-wound portion formed on the rear end portion in a longitudinal direction of the coil is greater than or equal to an outer diameter of the wire.
